# EUROPEAN PATENT APPLICATION

(11) **EP 1 391 204 A1**
(43) Date of publication of application: **25.02.2004**
(21) Application number: 02717122.2
(22) Date of filing: 12.04.2002
(51) Int. Cl.: A61K 31/661, A61P 25/00, A61P 25/16, A61P 25/28, A61P 43/00

(54) **NERVE CELL SURVIVAL PROMOTERS CONTAINING CYCLIC PHOSPHATIDIC ACID DERIVATIVE**

(30) Priority: 13.04.2001 JP 2001115925
(71) Applicant: Gencom Corporation, Machida-shi, Tokyo 194-8511 (JP)
(72) Inventor: MUROFUSHI, Kimiko, Saitama-shi, Saitama 336-0026 (JP); TIGYI, Gabor, Memphis, TN 38111 (US)
(74) Representative: Sternagel, Fleischer, Godemeyer & Partner Patentanwälte
(86) International application number: PCT/JP2002/003658
(87) International publication number: WO 2002/083148

(57) **Abstract**

An object of the present invention is to provide a novel medicament which is useful for treating and/or preventing a nerve disease by increasing the survival rate of nerve cells or promoting the elongation of nerve cells. According to the present invention, there is provided a medicament for promoting the survival of nerve cells, which comprises a cyclic phosphatidic acid derivative having a cyclic phosphoric acid structure at the sn-2 and 3 positions of glycerol.

## Description

### FIELD OF THE INVENTION

The present invention relates to a medicament which comprises a cyclic phosphatidic acid derivative, one of lysophospholipids. More particularly, the present invention relates to a medicament for promoting the survival of nerve cells, a medicament for promoting the elongation of nerve cells, and a medicament for treating and/or preventing a nerve disease, which comprise a cyclic phosphatidic acid derivative as an active ingredient.

### BACKGROUND ART

Glycerophospholipid, the main component of a biomembrane, has a glycerol skeleton which is coupled with two molecules of hydrophobic fatty acids and is bonded with a hydrophilic group such as cholin and ethanol amine via a phosphate group. A balance between the hydrophobic moiety and the hydrophilic moiety in the phospholipid is important for forming a stable lipid bilayer. On the other hand, lysophospholipid can not form a stable membrane structure and rather exhibits an action of a surface activity of destroying the same, because only one molecule of a fatty acid is bound thereto so that the lysophospholipid has relatively small hydrophobic portion as compared with hydrophilic groups.

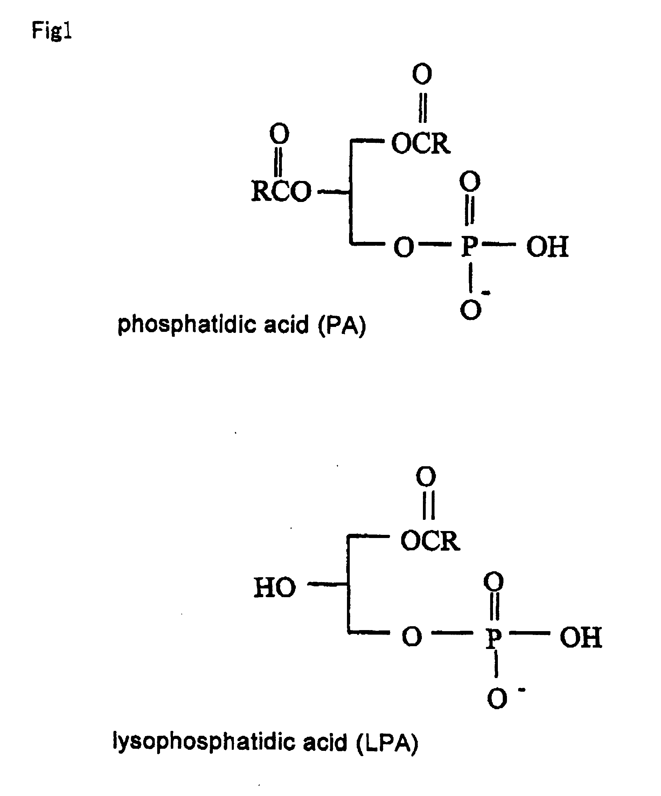

However, many lysophospholipids which exhibit specific physiological activities at a low concentrations have been recently found, and one example thereof is lysophosphatidic acid (LPA). LPA is one of phospholipids having the simplest structure, and can be discriminated from phosphatidic acid (PA) in that fatty acid either at sn-1 or -2 positions of glycerol is deacylated (See Fig. 1).

LPA exists in a living body at a very small amount (0.5% or less of a total cellular phospholipid). LPA was understood to be an intermediate product or a decomposed intermediate in the biosynthesis of a phospholipid. But in the latter half of 1970s, a substance which exists in plasma (Schumacher, K.A., et al., Thromb. Haemostas., 42, 631-640(1979)) or in a crude lecithin fraction from soy bean (Tokumura, A., et al., Lipids, 13, 468-472(1978)) and shows vasoconstrictive activity was identified to be LPA. Furthermore, it was also shown that a lipid growth factor in serum was LPA (van Corven, E., et al., Cell 59, 45-54(1989)), and LPA has attracted an attention as a physiologically active substance.

LPA has been demonstrated to have various physiological activities including cell proliferation promoting action (Fischer DJ.,et al., Mol Pharmacol, 54, 979-988 (1988)), promotion of infiltration of cancer cells (Imamura, F., et al.:Jpn.J.Cancer Res., 82, 493-496(1991); Imamura, F., et al.:Biochem. Biophys. Res. Commun., 193, 497-503(1993); and Imamura, F., et al.: Int. J. Cancer, 65, 627-632(1996)), inhibition of apoptosis (Umnaky, S.R., et al.: Cell Death Diff., 4, 608-616(1997)) and the like. Particularly, LPA is known to cause a recession of the neurodendrite of nerve cells (Tigyi, G., et al.:J. Biol. Chem., 267, 21360-21367(1992); Jalink, K., et al.: Cell Growth & Differ., 4, 247-255(1994); Jalink, K., et al.: J. Cell Biol., 126, 801-810(1994); and Tigyi, G., et al.: J. Nurochem., 66, 537-548(1996)). Also, LPA has been reported to induce opening release in PC12 cell, a nerve cell line (Shiono, S., et al.: Biochem. Biophys. Res Commun., 193, 663-667(1993)). Furthermore, in 1996, a gene of G protein-associated receptor (ventrlluar zone gene-1;vzg-1/edg-2) which is specifically expressed in a nerve epithelial cell layer (ventriluar zone, vz) has been cloned by Chun et al., and from the finding that lipid in serum is required for the morphological change of the cells which over-expresses said gene, it was revealed that its specific ligand was LPA (Hecht, J. H., et al. :J. Cell Biol. 135, 1071-1083(1996)). These observations suggest the importance of LPA signaling in a nerve system, and thus LPA is considered to play an important role in the development and differentiation of nerve.

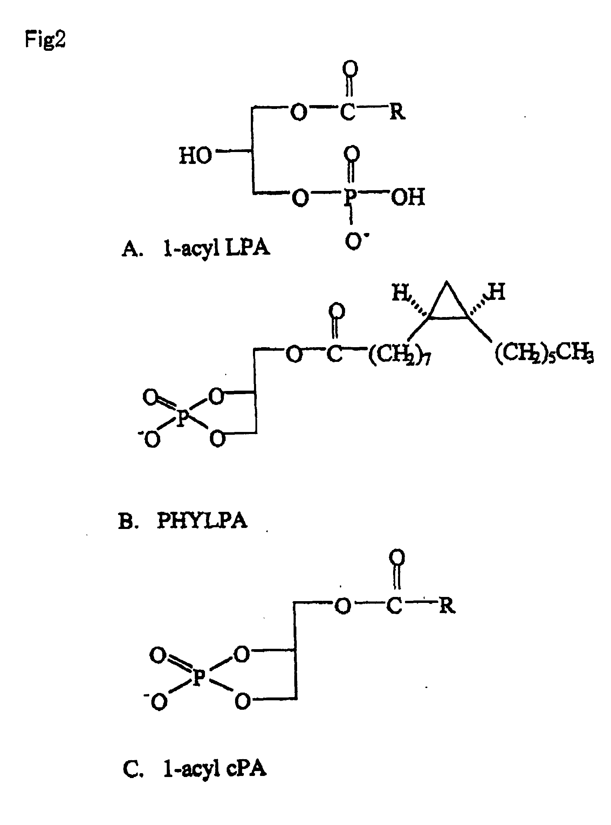

The present inventors have made a cellular biochemical analysis using *Physarum Polycephalum*, a myxomycete, as an experimental material. The myxomycete has been demonstrated to take a morphological change depending on variation of external environment and take a proliferation/differentiation with a remarkable change in the composition and metabolism of a biomembrane lipid. A novel lipid component which was isolated and identified from a haploid myxoamoeba in 1992 was analyzed structurally, and was confirm to be a substance which contains hexadecanoic acid having a cyclopropnane ring at the sn-1 position of a glycerol skeleton, and is esterified with phosphoric acid to form a ring at the sn-2 and 3 positions (Murakami-Murofushi, K., et al.: J. Biol. Chem.,267, 21512-21517(1992)). This substance is named PHYLPA, since it is a LPA analog derived from the Physarum (See Fig.2).

PHYLPA is obtained from a lipid fraction which inhibits the activity of DNA polymerase a in a eukaryotic cell and suppresses the growth of an animal cultured cell, and PHYLPA is confirmed to show these physiological activities. PHYLPA has a characteristic fatty acid, but the structural analogues wherein this fatty acid moiety is replaced with other common fatty acid moieties were organically synthesized, and their physiological activities were studied to reveal that they had the similar activities to PHYLPA (Murakami-Murofushi, K., et al.: Biochem.Biophys.Acta, 1258, 57-60(1995)). Therefore, it is assumed that the cyclic phosphoric acid structure at the sn-2 and -3 positions of the glycerol is essential for these physiological activities. The lipid having this structure is generally called a cyclic phosphatidic acid (cPA) (see Fig. 2).

It was confirmed that cPA was not a lipid peculiar to myxomytes, but exists widely in living world. For example, the cPA having a palmitic acid (C16:0) residue in the fatty acid portion was isolated and identified from human serum albumin-bonded lipid, suggesting the existence of a small amount of cPA bonded with myristic acid (C14:0) and stearic acid (C18:0) residues. The concentration of cPA in serum is expected to be about 10⁻⁷ M, which equals to about one tenth of the concentration of LPA in serum (Kobayashi.T., et al.; Life Science, 65, 2185-2191(1999)). Thereafter, it was confirmed that cPA is present in human serum and rabbit lacrimal gland liquid, as in the case of LPA (Liliom, K., et al.:Am. J. Physiol., 274, C1065-1074(1998)).

cPA has been reported to exhibit various physiological activities which are contrary or similar to those of LPA. For examples, cPA has been reported to inhibit a cell growth (Murakami-Murofushi, K., et al.: Cell Struct. Funct., 18, 363-370(1993)), to inhibit invasion of cancer cells (Mukai, M., et al.:Int.J.Cancer, 81, 918-922, 1999), and to form a stress fiber within cells (Fischer, D.J.,et al.: Mol.Pharmacol., 54, 979-988(1998)).

It is known that the nerve cells cannot survive if the supply of nerve growth factor (NGF) is stopped. For example, NGF is present at a high concentration in hippocampus (Hiroshi Hatanaka : Protein-Nucleic Acid-Enzyme, 35, 103-117, 1989). Further, it is also reported that fibroblast growth factor (FGF) which is an NGF-like cell growth factor, increases the survival rate of nerve cells of hippocampus and promotes the elongation of the neurite (Hiroshi Hatanaka : Biochemistry, 61, 1351-1365, 1989). However, there has been no report on the action of cPA on nerve cells.

### SUMMARY OF THE INVENTION

A problem to be solved by the present invention is to reveal the action of cPA on nerve cells as one of novel physiological activities of cPA and to provide a novel medicament which is useful for treating and/or preventing a nerve disease by increasing the survival rate of nerve cells or promoting the elongation of nerve cells.

In order to solve the above-described problem, the present inventors firstly tried to reveal the mechanism of cPA biosynthesis and then detect cPA in a calf brain. Furthermore, by using a primary culture system derived from a rat fetal brain, the present inventors tried to analyze the influence of cPA on the survival of nerve cells and the formation of neurite. From the result of these analyses, the inventors have found that, by revealing that cPA increases the survival rate of primary cultured nerve cells derived from a rat hippocampus and promotes the elongation of neurites, cPA can be a therapeutic agent useful for treating neuropathy, and thus the present invention has been completed.

Namely, according to the present invention, there is provided a medicament for promoting the survival of nerve cells, which comprises a cyclic phosphatidic acid derivative represented by the formula (I) below as an active ingredient: 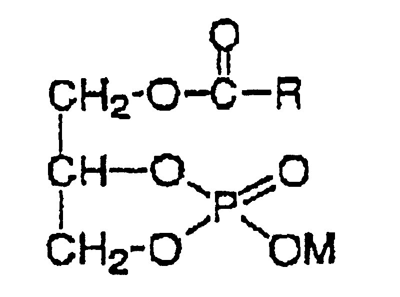 wherein R is a C₁₋₃₀ linear or branched alkyl group, a C₂₋₃₀ linear or branched alkenyl group, or a C₂₋₃₀ linear or branched alkynyl group, wherein these groups may contain a cycloalkane ring or an aromatic ring; and M is a hydrogen atom or a counter cation.

According to another aspect of the present invention, there is provided a medicament for promoting the elongation of nerve cells, which comprises a cyclic phosphatidic acid derivative represented by the formula (I) above as an active ingredient.

According to further another aspect of the present invention, there is provided a medicament for treating and/or preventing a nerve disease, which comprises a cyclic phosphatidic acid derivative represented by the formula (I) above as an active ingredient.

The nerve disease is selected, for example, from dementia, Alzheimer's disease, Alzheimer's senile dementia, amyotrophic lateral sclerosis, Parkinson's disease, cerebral stroke, cerebral infarction and head injury. The cyclic phosphatidic acid derivative represented by the formula (I) which is used in the present invention is preferably 1-oleoyl cyclic phosphatidic acid.

According to further another aspect of the present invention, there are provided a method for promoting the survival of nerve cells which comprises administrating a therapeutically effective amount of the cyclic phosphatidic acid derivative represented by the formula (I) above to a mammal including human; a method for promoting the elongation of nerve cells which comprises administrating a therapeutically effective amount of the cyclic phosphatidic acid derivative represented by the formula (I) above to a mammal including human; and a method for treating and/or preventing a nerve disease which comprises administrating a therapeutically effective amount of the cyclic phosphatidic acid derivative represented by the formula (I) above to a mammal including human.

According to further another aspect of the present invention, there are provided an use of the cyclic phosphatidic acid derivative represented by the formula (I) above in the production of a medicament for promoting the survival of nerve cells; an use of the cyclic phosphatidic acid derivative represented by the formula (I) above in the production of a medicament for promoting the elongation of nerve cells; and an use of the cyclic phosphatidic acid derivative represented by the formula (I) above in the production of a medicament for treating and/or preventing a nerve disease.

### BRIEF DESCRIPTION OF DRAWINGS

Fig.1 shows the structure of phosphatidic acid (PA) and lysophosphatidic acid (LPA).
Fig.2 shows the structure of lysophospholipids. "A" shows 1-acyl LPA, "B" shows PHYLPA, and "C" shows 1-acyl cPA.
Fig. 3 shows an overview of a method for extracting lipid components from a calf cerebrum.
Fig. 4 shows a method for purifying cPA by thin layer chromatography (TLC).
Fig. 5 shows the result by TLC analysis on a partially purified product derived from a calf cerebrum. The portion surrounded in pencil shows an area detected by the Primulin reagent. (a) shows the detection of cPA in the extract, and (b) shows the determination of the cPA level in the extract.
Fig. 6 is a diagram which shows the states of nerve cells at 48 hours after addition of BSA (a), cPA (b) or NGF (c).
Fig. 7 is a graph showing the influence of cPA on the nerve cell density. (a) shows the relationship between the cell density and the survival rate. (b) shows the detetmination of an optimal cell density.
Fig. 8 is a graph showing the influence of cPA on the survival rate of nerve cells.
Fig. 9 is a diagram showing nerve cells as data for determining an optimal cPA level for increasing the cell survival rate.
Fig. 10 is a graph showing the influence of cPA on the elongation of neurite. The vertical axis is shown by a ratio relative to the length of a control nerve cell after 24 hours (a) or 12 hours (b), which is assumed to be 1.
Fig. 11 is a graph showing the relationship between the cPA level and the ratio of cells having neurite.
Fig. 12 is a diagram of nerve cells, which shows the relationship between the elongation of neurite and the cPA level.
Fig. 13 is a graph showing the influence of the P13K inhibitor on the action of increasing the survival rate by cPA. Wortmannin (30nM), LY294002 (10µM), cPA (1 µM)

### BEST MODE FOR CARRYING OUT THE INVENTION

Hereafter, embodiments of the present invention will be described in detail.

A medicament of the invention can be used for increasing the survival rate of nerve cells, for promoting the elongation of nerve cells, and for treating and/or preventing nerve diseases, and the medicament comprises a cyclic phosphatidic acid derivative represented by the formula (I) below as an active ingredient: 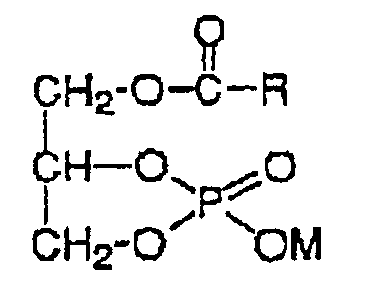 wherein R is a C₁₋₃₀ linear or branched alkyl group, a C₂₋₃₀ linear or branched alkenyl group, or a C₂₋₃₀ linear or branched alkynyl group, wherein these groups may contain a cycloalkane ring or an aromatic ring; and M is a hydrogen atom or a counter cation.

Examples of the C₁₋₃₀ linear or branched alkyl groups represented by the substituent R in the formula (I) include a methyl group, an ethyl group, a propyl group, a butyl group, a pentyl group, a hexyl group, a heptyl group, an octyl group, a nonyl group, a decyl group, a pentadecyl group, and an octadecyl group.

Examples of the C₂₋₃₀ linear of branched alkenyl group represented by the substituent R include an allyl group, a butenyl group, an octenyl group, a decenyl group, a dodecadienyl group, and a hexadecatrienyl group. More specifically, the examples include 8-decenyl group, 8-undecenyl group, 8-dodecenyl group, 8-tridecenyl group, 8-tetradecenyl group, 8-pentadecenyl group, 8-hexadecenyl group, 8-heptadecenyl group, 8-octadecenyl group, 8-icocenyl group, 8-dococenyl group, heptadeca-8,11-dienyl group, heptadeca-8,11,14-trienyl group, nonadeca-4,7,10,13-tetraenyl group, nonadeca-4,7,10,13,16-pentaenyl group, and henicosa-3,6,9,12,15,18-hexaenyl group.

The examples of the C₂₋₃₀ linear or branched alkynyl group represented by the substituent R include 8-decynyl group, 8-undecynyl group, 8-dodecynyl group, 8-tridecynyl group, 8-tetradecynyl group, 8-pentadecynyl group, 8-hexadecynyl group, 8-heptadecynyl group. 8-octadecynyl group, 8-icocynyl group, 8-dococynyl group, and heptadeca-8,11-diynyl group.

The examples of the cycloalkane ring which may be contained in the above described alkyl group, alkenyl group or alkynyl group include, for example, a cyclopropane ring, a cyclobutane ring, a cyclopentane ring, a cyclohexane ring, and a cyclooctane ring. The cycloalkane ring may contain one or more hetero atoms, and examples thereof include an oxylane ring, an oxetane ring, a tetrahydrofuran ring, and an N-methylprolidine ring.

The examples of an aromatic ring which may be contained in the above described alkyl group, alkenyl group or alkynyl group include, for example, a benzene ring, a naphthalene ring, a pyridine ring, a furan ring, and a thiophene ring.

Accordingly, in the case where the substituent R is an alkyl group substituted with a cycloalkane ring, the examples include a cyclopropylmethyl group, a cyclohexylethyl group, and an 8,9-methanopentadecyl group.

In the case where the substituent R is an alkyl group substituted with an aromatic ring, the examples include a benzyl group, a phenetyl group, and a p-pentylphenyloctyl group.

M in the cyclic phosphatidic acid (cPA) derivative represented by the formula (I) is a hydrogen atom or a counter cation. In the case where M is a counter cation, examples thereof include an alkali metal atom, an alkali earth metal atom, and a substituted or unsubstituted ammonium group. The alkali metal atom includes, for example, lithium, sodium and potassium. The alkali earth metal atom includes, for example, magnesium and calcium. The substituted ammonium group includes, for example, a butylammonium group, a triethylammonium group and a tetramethylammonium group.

As a specific example of the cPA represented by the formula (I) which is used in the present invention, an oleoyl cPA is particularly preferable.

The cPA derivative represented by the formula (I) can be chemically synthesized according to the methods disclosed in, for examples, Japanese Patent Laid-open Publications JP-A-5-230088, JP-A-7-149772, JP-A-7-258278, and JP-A-9-25235.

Alternatively, the cPA derivative represented by the formula (I) can also be synthesized by reacting the lysophospholipid with phospholipase D according to the method described in Japanese Patent Application No.367032/1999. The lysophospholipid used here is not limited, so far as it can be reacted with phospholipase D. Many types of lysophospholipids are known, including those which are different in fatty acid and molecular species which have an ether or vinylether bond. They are available in the market. As for the phospholipase D, those derived from a higher plant such as cabbage and peanut or from a microorganism such as *Streptomyces chromofuscus* and *Actinomadula sp*. are available in the market, cPA can be highly selectively synthesized with the enzyme derived from *Actinomadula sp*. No.362 (Japanese Patent Laid-open Publication JP-A-11-367032). Any condition may be available without limitation for reacting the lysophospholipid with the phospholipase D, as far as it allows the enzyme to exhibit the activity. The reaction of the lysophospholipid with the phospholipase D is carried out, for example, in an acetate buffer (around pH 5-6) containing calcium chloride at room temperature to a warmed temperature (preferably about 37°C) for around 1-5 hours, although the condition of the reaction is not particularly limited so far as the condition allows the expression of the enzyme activity. The thus produced cPA derivative may be purified by extraction, column chromatography, thin layer chromatography (TLC) or the like according to a conventional method.

The cyclic phosphatidic acid derivative, which is used as an active ingredient in the present invention, can increase the survival rate of nerve cells and promote the elongation of nerve cells. Thus, according to the invention, there is provided a medicament for treating and/or preventing a nerve disease which comprises the cyclic phosphatidic acid derivative as an active ingredient. The nerve disease in this specification is preferably a brain nerve disease (neuropathy in a brain), and specific examples thereof include a nerve denaturation disease, cerebral stroke, cerebral infarction, dementia, and head injury. The nerve denaturation disease herein is a disease where nerve cells contract or denature to disappear, and examples thereof include Alzheimer's disease, Alzheimer's senile dementia, amyotrophic lateral sclerosis, and Parkinson's disease.

The medicament of the present invention is preferably provided in the form of a pharmaceutical composition which comprises one or more pharmaceutically acceptable additives and the cPA derivative represented by the formula (I) as an active ingredient.

The medicament of the present invention can be administered in various forms, and preferably has a form capable of passing through a blood-brain barrier, since its main active site is brain. Such suitable dosage forms may be peroral or parenteral (for examples, intravenous, intramuscular, subcutaneous or intracutaneous injection, rectal dosage, and permucosal dosage). Examples of the pharmaceutical composition suitable for peroral dosage include a tablet, a granule, a capsule, a powder, a solution, a suspension, and a syrup, Examples of the pharmaceutical composition suitable for parenteral dosage include an injection, an infusion, a suppository, and a percutaneous absorption agent. The dosage form of the medicament of the present invention is not limited to these. Furthermore, the medicament of the present invention can also be made into sustained release formulations by publicly known methods.

The type of the pharmaceutical additives used for producing the medicament of the present invention is not particularly limited, and can be suitably selected by a person skilled in the art. For examples, one can use an excipient, a disintegration agent or a disintegration auxiliary agent, a binder, a lubricant, a coating agent, a base, a solvent or a solubilizer, a dispersant, a suspension agent, an emulsifier, a buffer, an antioxidant, an antiseptic, an isotonic agent, a pH adjusting agent, a solving agent, and a stabilizer. Individual ingredients which are used for the above purposes are well known to a person skilled in the art.

Examples of the pharmaceutical additives usable for preparing a peroral preparation include an excipient such as glucose, lactose, D-mannitol, starch and crystalline cellulose; a disintegration agent or a disintegration auxiliary agent such as carboxymethyl cellulose, starch and carboxymethyl cellulose calcium; a binder such as hydroxypropyl cellulose, hydroxypropyl methylcellulose, polyvinyl pyrrolidone, and gelatin; a lubricant such as magnesium stearate and talc; a coating agent such as hydroxypropyl methylcellulose, white sugar, polyethylene glycol and titanium oxide; a base such as Vaseline, liquid paraffin, polyethylene glycol, gelatin, kaolin, glycerin, purified water, and hard fat.

Examples of the pharmaceutical additives which can be used for preparing an injection or an infusion preparation include a solvent or a solubilizer which can be used for an aqueous injection or a use-time dissolution type injection such as injection distilled water, physiological saline, and propylene glycol; an isotonic agent such as glucose, sodium chloride, D-mannitol, and glycerin; and a pH adjusting agent such as an inorganic acid, an organic acid, an inorganic base and an organic base.

The medicament of the present invention can be administered to a mammal including human.

The dose of the medicament of the present invention should be increased or decreased according to the conditions such as age, sex, body weight, symptom of a patient, and dosage route. The dose of the active ingredient per day for an adult is generally 1µg/kg to 1,000mg/kg, and preferably 10µg/kg to 100mg/kg. The medicament of the dose as mentioned above may be administered once a day, or may be dividedly administered a few times (for example, about 2-4 times) a day.

The medicament of the present invention may be used in combination with another medicament which is effective for treating or preventing a nerve disease, a nutrient for supplying brain nerve with energy, or the like.

As is obvious from Example 1 as described later, cPA itself is a substance which exists in brain of mammals, and is considered to be safe to a living body.

All the disclosures in Japanese Patent Application No. 115925/2001, which the present application claims priority based on, shall be disclosed herein by reference.

The present invention will be described in detail with reference to the following Examples, but the present invention is not limited by the Examples.

### EXAMPLES

### Example 1: Biosynthesis of cPA using phospholipase D (PLD)

In Example 1, it was revealed that cPA can be generated from lysophosphatidyl cholin (LPC) using an actinomyces-derived PLD, and that an enzyme for generating cPA exists in the brain of mammalian.

### (A) Material and method

### (A-1) Experiment material

PLD derived from an actinomyces, *Streptomyces chromofuscus* (*S.chromofuscus*), and PLD derived from cabbage were purchased from Sigma. PLD derived from *Actinomadura sp*. No. 362 (*A, sp,* No.362) was purchased from the Meito Sangyo. 1-oleoyl LPC and lysophosphatidylserine (LPS) were purchased from Avanti Polar lipid, INC. Lysophosphatidylethanolamine (LPE) was purchased from Doosan Serdary Res. Lad. 1-alkyl lysophosphatidylcholine (1-alkyl LPC) and 1-alkenyl phosphatidylcholine plasmalogen (1-alkenyl LPC) were purchased from Sigma. Oleoyl cPA which was synthesized according to the method described in Kobayashi, S., et al.: Tetrahedron Lett., 34, 4047-4050(1993) was also used.

### (A-2) Synthesis of 1-NBD-LPC

A fluorescence labeled LPC was prepared as a substrate for determining the cPA generating activity. Namely, by using 1-hexadecanoyl-2-(12-(7-nitrobenz-2-oxa-1,3 -diazol-4-yl))-sn-glycero-3-phosphocholin (2-NBD-HPC; made by Avanti Polar lipids, INC.) as a starting material, a fatty acid only at 1-position was deacylated with a lipase (derived from *Rhizopus delemer,* made by Seikagaku Kogyo), then a fluorescence labeled acyl group at 2-position was displaced to 1-position in Tris-HCl buffer (pH=9), and the product was purified by high performance liquid chromatography (HPLC) to obtain 1-NBD-LPC.

### (A-3) Assay of cPA generating activity

As the substrate, a mixture of 1-NBD-LPC and egg yolk derived LPC at the ratio of 1:99 was used (1% NBD-LPC). The enzyme assay was carried out in 100mM acetate buffer (pH=5.6) containing 10mM calcium chloride in the presence of 100µM of 1% NBD-LPC. For the enzyme source, 2.2µg/ml of PLD derived from an actinomyces, *S.chromofuscus*, or 1.4µg/ml of PLD derived from an actinomyces, *A. sp*. No.362, was used. The reaction was carried out at 30°C (in the case of *S.chromofuscus*) or at 37°C (in the case of *A. sp*. No. 362). At the end of the reaction, 0.3 times volume of 0.1M citric acid solution was added to the reaction solution to make it acidic, and then 5.4 times amount of a chloroform:methanol (2:1) mixed solution was added, followed by centrifuging (1,400 x g, 5 minutes) to extract a lipid in the lower layer. The same extraction with the chloroform:methanol (2:1) mixed solution was repeated once again, and the obtained lower layers were mixed and concentrated to dry under a nitrogen stream. The thus obtained lipid was dissolved again in a small amount of the chloroform:methanol (2:1) mixed solution, and was spotted on a Silica Gel 60F thin layer chromatography plate (TLC; made by E. Merck). Using a developing solvent: chloroform/methanol/acetic acid/5% aqueous sodium bisulfite solution (100:40:12:5), the lipid was separated, and the intensity of each fluorescent spot was quantified by a fluoroimage analyzer, FLA-2000 (made by Fuji Photo Film).

### (A-4) Structural analysis by ESI-MS/MS

By using an apparatus manufactured by connecting Quattro II (made by the Micromass), which was a Tandem quadrupole type mass spectrometer equipped with an electrospray type ion source, with HPLC, a sample was analyzed in an anionic mode. By using Hewlett Packerd model 1050 HPLC pump (made by Hewlett Packerd), the sample was eluted with an acetonitrile:methanol (1:1) mixed solution at a flow rate of 5µg/min, 3-5µl of the sample, which contained lipid dissolved to be a concentration of 10-50 pmol/µl in the acetonitrile/methanol (1:1) mixed solution containing 0.1% ammonium formate, was injected. Formic acid and ammonia work as a proton donor or acceptor when the sample was ionized, respectively. The interface between the HPLC and the MS was maintained at 80°C, and nitrogen gas for purging the solvent was set at a pressure of 40 psi and a flow rate of 0.4 l/min. In the MS analysis, a molecular ion was monitored at a cone voltage of -30eV. In the MS/MS analysis, a fatty acid was monitored at a cone voltage of -90eV, and and phosphoric acid was monitored at a cone voltage of -170eV. In the MS/MS analysis, a technique was combined where a high pressure is locally formed by introducing an inert gas, and daughter ions were generated by collision induced dissociation (CID) of molecule ions. Argon (pressure 3.0-4.5e⁻⁴ Torr) was used as the collision gas, and the collision energy was set to be -50eV.

### (A-5) Formation of stress fiber in NIH-3T3 by addition of various lysophospholipids

NIH-3T3, a mouse-derived fibroblast cell, was incubated in a Dulbecoo's Modified Eagle's Medium (DMEM) containing 10% fetal bovine serum (FBS; made by Moregate), and was used in the Experiment. 2.5x10⁴ cells of the NIH-3T3 were inoculated in a petri dish (10cm in diameter) paved with cover glasses of 22mm in diameter, and the medium was replacing with a FBS-free medium after 24 hours, and the cells were incubated in a serum starvation state for 48 hours. 10µM of the lysophospholipid was added, and the cells were incubated at 37°C for 30 minutes for stimulation. Then, the cells were fixed in the Dalbecco's PBS containing 3.7% of paraformaldehyde and 0.1% of Triton X-100 at room temperature for 10 minutes. Thereafter, the cells were stained with 5 units/ml of rhodamine phalloidin (made by Funakoshi) at 37°C for 1 hour. The cover glasses were washed with PBS thrice, and observation was carried out by a cofocus laser microscope TCS NT Control laser Scanning Microscope (made by Leica).

### (A-6) Assay of cPA generating activity in rat brain

Male 4-weeks old Sprague-Dawley rats were employed for subjects. The rats were anesthetized with ether and decapitated to separate their whole brains, which were preserved at -80°C. Either left or right half side (about 0.8g) of the rat whole brains thus preserved was added with 10 times volume of 0.32M sucrose solution, and was homogenized twice for 20 seconds by the Polytron Homogenizer (made by Polytron) at a power control of 7. Twice volume of Hepes buffer was added thereto to prepare a homogenate solution. The assay of cPA generating activity was carried out in the following solution composition. Namely, 1%NBD-LPC 40nmol or a mixture (120nCi) of ¹⁴C-LPC which was radiation-labeled at the carbon in the carboxyl group of the fatty acid at 1-position, and an unlabeled LPC at a ratio of 2:55 was used as a substrate, and it was reacted in 100µl of the homogenate solution in the presence of 450µM of sodium oleate at 37°C. As for process after the completion of the reaction, the conditions for the actinomyces PLD assay were applied.

### (B) Results

### (B-1) Generation of LPA/cPA by actinomyces PLD

It was studied whether the generation of cPA could be found or not by using two types of actinomyces-derived PLDs which were different in the intensity of phosphate displacement activity and using 1%1-NBD-LPC as a substrate. In the case where the *S.chromofuscus*-derived PLD was used, the reaction of 20 minutes yielded only 1-NBD-LPA as the main product. On the other hand, in the case where the A.sp.362-derived PLD which was considered to have a high phosphate displacement activity was used, a product different from LPA was mainly obtained, and the Rf value of this compound coincided with that of the organic-synthesized cPA standard (oleoyl cPA). In order to confirm the difference between these reactions, the enzyme level dependency and the reaction time dependency were studied respectively. In the case where the *S*.*chromofuscus*-derived PLD was used, only the generation of LPA was observed with decrease of LPC which is the substrate. In contrast, in the case of the A.sp.362-derived PLD was used, only increase of product corresponding to cPA was observed, and almost no generation of LPA was observed. These observations shows that different types of LPDs generates different products regardless of the same substrate. In the case where the cabbage-derived PLD, which have been well analyzed enzymologically, was used, both LPA and cPA were generated at a rate of about 6:4.

Here, the A.sp.362-derived PLD, which generated a cPA-corresponding compound, was further studied with respect to substrate apecificity. By using each 100µM of 1-acyl LPC, 1-alkyl LPC, 1-alkenyl LPC, LPS, and LPE as a substrate, reaction was carried out according to the standard PLD assay condition. The product from respective substrates was separated by TLC, and the spots corresponding to LPA and cPA were collected, and the generation amount was determined by phosphor quantification. Almost no generation of LPA was observed for both substrates. In the case where LPC, 1-alkyl LPC or 1-alkenyl LPC was used as a substrate, cPA generated time-dependently, while cPA was not generated when LPS and LPE were used as the substrate. From these results, it was showed that the A.sp.362-derived PLD generated cPA effectively from the lysophospholipid having a choline at the polar group moiety. Additionally, alkyl and alkenyl type LPC were also found to be able to be a substrate for cPA generation.

### (B-2) Structural analysis of a reaction product by A.sp.362-derived PLD

Mass spectrometry was utilized to perform structural analysis of the main product (a compound having the same Rf value as that of cPA) obtained by reacting, a substrate, 1-oleyl LPC having oleic acid as the fatty acid at 1-position, with A.*sp*.362-derived PLD. Firstly, in order to set the condition for the mass spectrometry, organically synthesized 1-oleoyl cPA was analyzed as a standard by ESI-MS/MS in an anionic mode. As a result, a peak of m/z 417 corresponding to the molecular ion [M-H]⁻ of the 1-oleoyl cPA was observed under the above described condition. When the PLD reaction product was analyzed under the same condition, a strong peak of m/z 417 was similarly observed. In order to obtain more structural information, tandem mass spectrometry (MS/MS) by in-source fragmentation was carried out on a group of peaks of the molecular ion. As a result of daughter scanning using a molecular ion m/z 417 of the standard cPA as the parent ion, several characteristic ion peaks were generated. It was understood that m/z 281, m/z 153, and m/z 79 were attributed to C₁₇H₃₃COO⁻, [M-C₁₇H₃₃CO]⁻, and PO₃⁻ respectively. On the other hand, the m/z 417 peak obtained by the PLD reaction was similarly analyzed by MS/MS, and as a result, a group of the identical characteristic fragment peaks was observed. From the aforementioned result, it was confirmed that the compound generated by the *A*.*sp*.362-derived PLD reaction was cPA.

### (B-3) Formation of stress fiber in NIH-3T3 cell by PLD reaction product

The product obtained by the A.sp.362-derived PLD reaction was studied with respect to its biochemical activity in order to confirm further that it was a compound which is identical to cPA. Namely, the activity of forming actin stress fiber in a fibroblast cell, one of the physiological acteivities of cPA, was studied. To NIH-3T3, i.e. a fibroblast cell line derived from mouse, in the subconfluent state in the serum starvation state, each 10µM of LPA, a chemically synthesized PHYLPA, or cPA, i.e. the PLD reaction product was added at 37°C for 30 minutes, and then actin stress fiber was stained with Rhodamine phalloidin, and observation was carried out. In the control cells without addition of the lipid, no formation of stress fiber was observed, while the formation of stress fibers was observed in the case of addition of either one of the three types of lipids.

### (B-4) Generation of cPA in rat brain

Possibility of existence of cPA generating activity in rat brain was studied. As the result of studies under several different conditions, finally homogenate was prepared with an aqueous sucrose solution (0.32M), and the activity was determined in the Hepes buffer (pH 7.2) in the presence of oleic acid (450µM). As a result, cPA corresponding spots were confirmed after incubation at 37°C for 60 minutes.

### (C) Conclusion

From the above results, it was showed that cPA could be generated by a specific enzyme among phospholipid hydrolases generally called PLD. It was revealed that even using PLDs available in the market as purified products, if its enzyme source was different, a different product was obtained when LPC was used as a substrate.

The finding of Example 1 that the enzymatic activity for generating cPA from LPC was detected in the rat brain homogenate, shows that phosphatidyl group displacement reaction contributes positively to the production of cPA which is a physiologically active lipid. A mammalian brain has been demonstrated to contain cPA and also have a relatively high PLD activity. The substrate LPC is scarcely detected in brain under a normal physiological condition, but is considered to be generated through activation of a certain type of PLA₂.

It is useful for producing cPA-structural homologues that cPA can be prepared effectively by the use of the actinomyces *A.sp*.362-derived PLD. In the preparation method using such an enzyme, cPA can be prepared from 1-alkenyl LPC. 1-alkenyl LPA is detected in the injury of a rabbit cornea, has an activity for proliferating cells, and is involved in healing of a wound. In addition, the corresponding LPAs/cPAs can be prepared from LPCs which are different in fatty acid.

### Example 2: Detection of cyclic phosphatidic acid from a calf cerebrum

In Example 2, for the purpose of the detection of cPA in a mammalian brain, it was confirmed that cPA was present in a calf cerebrum.

### (A) Material and method

### (A-1) Experiment material

Oleoyl-cPA (an organically synthesized product) used as a standard was organically synthesized according to the method as described in Kobayashi, S. et al.: Tetrahedron Lett., 34, 4047-4050(1993). The calf cerebrum was purchased from Tokyo Shibaura Zohki KK.

### (A-2) Extraction of lipid components from a calf cerebrum

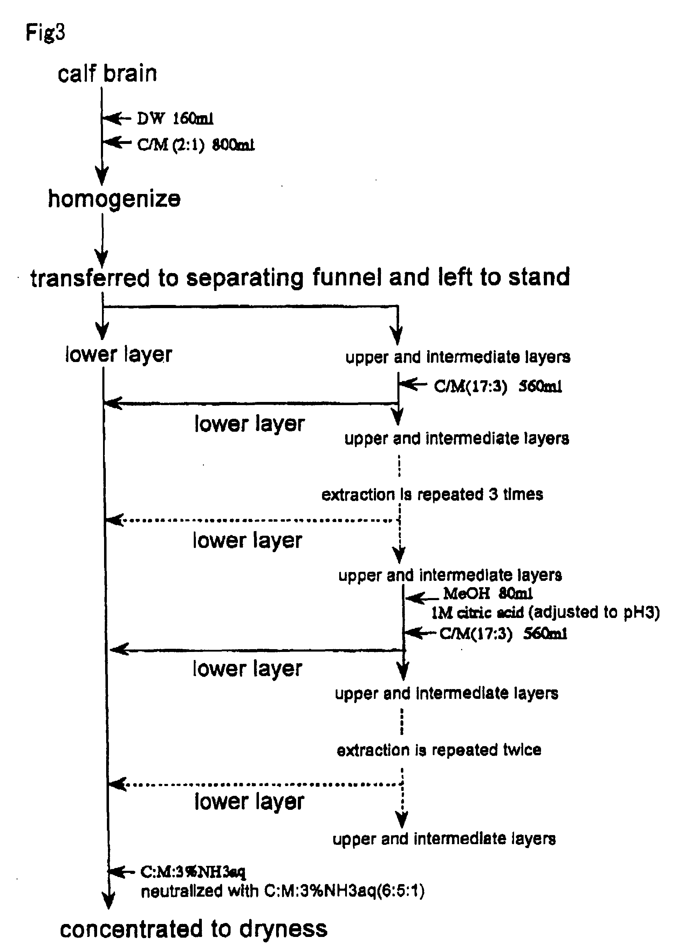

The cPA was confirmed to be present in a calf cerebrum by using the same as the start material. In Fig. 3, the procedure is briefed. Namely, 160ml of water and 800ml of a chloroform-methanol mixture (2:1) were added to 40g of a part of a calf brain. After homogenization, the mixture was put in a separation funnel, stirred, and left to stand at room temperature. The lower layer (chloroform layer) was isolated. The remaining upper and middle layers were extracted four times with 560ml of a chloroform-methanol mixture (17:3)(v/v), and the lower layer was separated. Subsequently, 80ml of methanol and 1M citric acid were added to the upper and middle layers to adjust about pH 3, and the mixture was left under stirring for 30 minutes. Then, it was extracted three times with 560ml of a chloroform-methanol mixture (17:3) to isolate the lower layer. All the chloroform layers obtained were mixed, neutralized with a chloroform/methanol/3% aqueous ammonia mixture (6:5:1) to be pH 7, and dried in a nitrogen evaporator. The extract was suspended again in a chloroform-methanol mixture (1:1) and used as a lipid fragment for purification as described below.

### (A-3) Purification by thin layer chromatography (TLC)

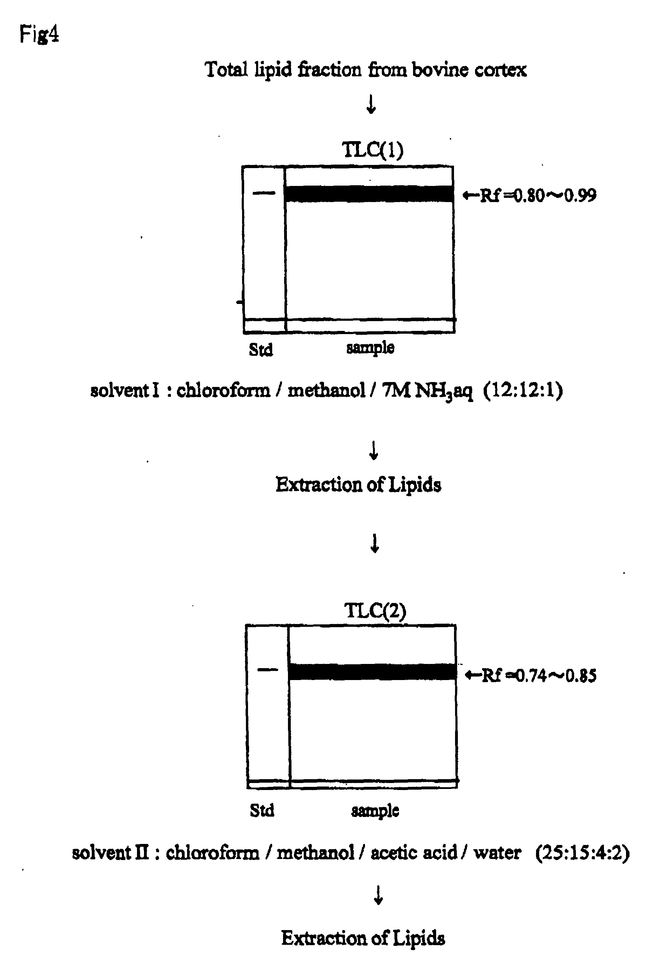

The lipid extracts derived from a calf cerebrum was sequentially separated by the thin layer chromatography using the following developing solvents system (Fig. 4). For separation, Silicagel 60 TLC plate (E. Merck No.5745; made by E. Merck) was used.
I: chloroform/methanol/7M aqueous ammonia (12:12:1)(v/v)
II: chloroform/methanol/acetic acid/water (25:15:4:2)(v/v)

The organically synthesized oleoyl-cPA as the standard was placed at the edge of a plate. After development, only the standard part was allowed to develop a color with the Dittmer reagent, which is a phosphorus-specific coloring reagent. For the plate on a part of which the extract was placed, the area corresponding to the standard's Rf value was gathered. From silica powder, lipids were extracted with chloroform-methanol mixture solvents (1:2), (1:1), and (2:1) in this order twice for each solvent. All the extract solutions were mixed, and the solvents were removed by nitrogen gas. The residual material was suspended again in a chloroform-methanol mixture solvent (1:1), and silica gel was removed. The purified preparation was analyzed by a two-dimensional TLC using the following developing solvents. For separation, Silica gel 60 TLC plate (B. Merck No.5721; made by E. Merck) was used.
III: chloroform/methanol/water (60:40:9)
IV: chloroform/methanol/acetic acid/acetone/water (10:2:2:4:1)

The Primulin reagent capable of coloring lipids was sprayed for the coloring of the lipid on the plate. Then, fluorescent spots were detected with an UV lamp and marked in pencil. Thereafter, the Dittmer reagent was sprayed, and the spots containing phospholipid were detected and quantified.

### (B) Result

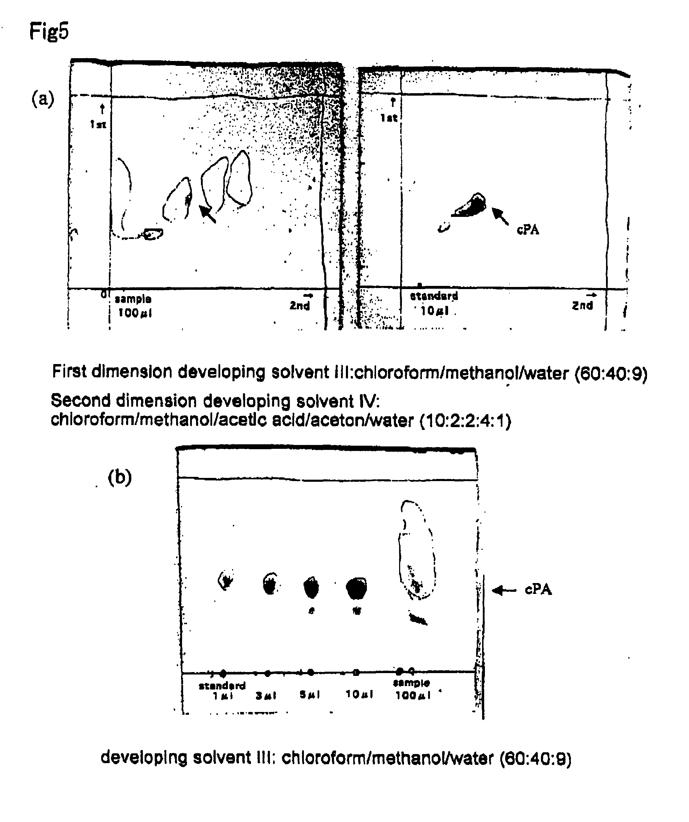

The purification started with 40g by wet weight of a calf cerebrum. After repeatedly performing extraction with a chloroform-methanol mixture, about 6.95g of a crude lipid fraction was extracted. 1g of this extract was dissolved in 2ml of a chloroform-methanol mixture (1:1), and 1ml of this solution was spotted to 2mm thick TLC plate. As for TLC, the areas having respective Rf values of 0.80-0.98 and 0.74-0.85 were gathered by using the above described developing solvents (I) and (II) in this order. The partially purified samples were analyzed by two-dimensional TLC using the above described developing solvents (III) and (IV). As a result, the spot showing the Rf value which is the same as that of the standard cPA was confirmed (Fig. 5(a)).

Color development with the Dittmer reagent, which is a phospholipid-specific coloring reagent, through the TLC using the above described developing solvent (III), was compared with a control curve which was prepared by using known amounts of the standard cPA, and the level of cPA contained in the extract was determined (Fig. 5(b)). From an approximate calculation for the amount of the above described cPA, it can be summarized that about 2.1mg of cPA was purified from 40g by wet weight of a calf cerebrum.

### (C) Conclusion

Generally, lipid is known to occupy 5-15% of wet weight of a mammalian brain and 65% of dry weight of a mammalian brain. Thus, it can be mentioned that the brain is one of organs containing a largest amount of lipid. In Example 2, about 7g of total lipid was extracted from 40g of a calf cerebrum. Further, about 2.1mg of lipid corresponding to cPA was finally detected. This value corresponds to 0.1% or less of the weight of total lipid in a brain, even if a loss during the purification is taken into account. Since each of phosphatidyl ethanolamine (PB) and phosphatidyl cholin (PC), main phospholipids in a rat brain, has a ratio of about 20% relative to the total lipid, cPA is a minor component, the amount of which is about 1/200 or less of these phospholipids. However, it has been demonstrated that cPA exists in a mammalian brain.

### Example 3: Action of a cyclic phosphatidic acid on the primary cultured nerve cells derived from a rat fetal brain (the influence on the survival rate and the neurites)

In Example 3, an influence of cPA on the survival of neurocytes and the formation of neurites was analyzed by using primary cultured nerve cells derived from a rat fetal brain. As a result, the action of cPA on the nerve cells (improvement of the survival rate of the nerve cells and promotion of the elongation of neurite) was demonstrated as a novel physiological activity cPA.

### (A) Material and method

### (A-1) Preparation and culturing of the primary cultured nerve cells derived from a hippocampus of a rat fetal brain

Sprague-Dowley rat with 16 days of pregnancy were used as material. The rat were anesthetized with ether, from which the fetal was taken out. The whole brain was taken out under a stereomicroscope, and a hippocampus inside the cerebral cortex was picked out. The picked out hippocampus was collected in a 15ml centrifuging tube, and 0.5ml of 2.5% trypsin and a culture solution was added to be a 5ml solution. The mixture was incubated at 37°C for 15 minutes and then centrifuged at 3,000rpm for 15 minutes. The supernatant was removed, 5ml of the culture solution was added, and the mixture was centrifuged at 3,000rpm for 15 minutes. The procedure was additionally repeated twice. After adding 3-5ml of the culture solution, the resultant was pipetted with a Pasteur pipette and then with an injection needle (TERUMO NEEDLE (0.70x38mm); made by Terumo). The cells, which were finally dissociated through a cell strainer (FALCON Cell Strainer 70µm; made by FALCON), were primarily cultured on a plate. A culture liquid and a method for coating a plate which allow the nerve cells to be cultured without aggregation, were studied.

The cPA derivative used in this test was an oleoyl cPA which was synthesized according to the method as described in Example 1 by using oleoyl LPC as a substrate and using the PLD derived from actinomyces *A*.*sp*.No.362 as an enzyme source.

### (A-2) Determining the survival percentage of neurocytes

The cells were primarily cultured according to the method as described in (A-1). At the same time when the cells were inoculated on the plate, the oleoyl cPA (5µm) was added to nerve cells each having different density from each other. After a certain period of time, cells were photographed with a phase contrast microscope (magnification: 20, five photographs per well on a 24 well plate), and the survival of the cells was judged morphologically to calculate the survival rate.

Further, in order to determine an optimal cPA level to increase the cell survival rate, the cells were cultured at a cell density (3.0x10⁵ cells/cm²) at which the highest survival rate of nerve cells was observed. At the same time when the cells were inoculated on the plate, each of 0.5, 1.0, 5.0, or 10.0µM of oleoyl cPA was added to the cell culture solution. The cells were photographed as mentioned above and the survival of the cells was judged on the photographs.

### (A-3) Measurement of the elongation of neurites

In order to study the influence of cPA on the elongation of neurites, the cells were primarily cultured according to the method as described in (A-1), and were photographed at 12, 18 and 24 hours after the start of the culture. The average length of neurites per cell and the number of cells having the neurite at respective time points were analyzed by an image analysis software (NIH Image 1.62).

### (A-4) Analyze of an intracellular signal transduction system

Wortmannin (made by Sigma) and LY294002 (made by Sigma), which are inhibitors against phosphatidyl inositol 3-kinase (PI3K), were dissolved in DMSO at the concentration of 10mM and 50mM respectively, and diluted with PBS. Each of the solutions was added to the cell culture solution at the respective final concentrations of 30nM and 10µM. The inhibitors were added to the cell culture solution at 2 hours prior to the addition of cPA, to carry out pretreatment. The cells were photographed at 48 hours after the addition of cPA, and the survival of the cells was morphologically judged on judged on the photographs to calculate the survival rate.

### (B) Result

### (B-1) Establishment of an experimental system for nerve cell primary culture conditions

As a preliminary test, various culture conditions were studied, and tests were performed on coatings and culture solution that allowed nerve cells to adhere to the plate. As the result, it was found that the Ham's F-12 was suitable as the culture solution, and poly-L-lysine or poly-L-ornithine was suitable for the coating of the plate. Therefore, a plate coated with poly-L-lysine (IWAKI MICROPLATE; made by IWAKI) was used hereinafter. A serum-free culture was used because cPA or LPA might have existed already in serum. The N1 supplement (containing insulin 5µg/ml, transferin 5µg/ml, progesterone 20nM, putrescine 100µM, and sodium selenite 30nM; made by Sigma) was added as a growth-promoting factor (serum-free culture auxiliary factor) instead of serum.

### (B-2) Action of cPA on the survival rate of primary culture nerve cells derived from a rat fetal hippocampus

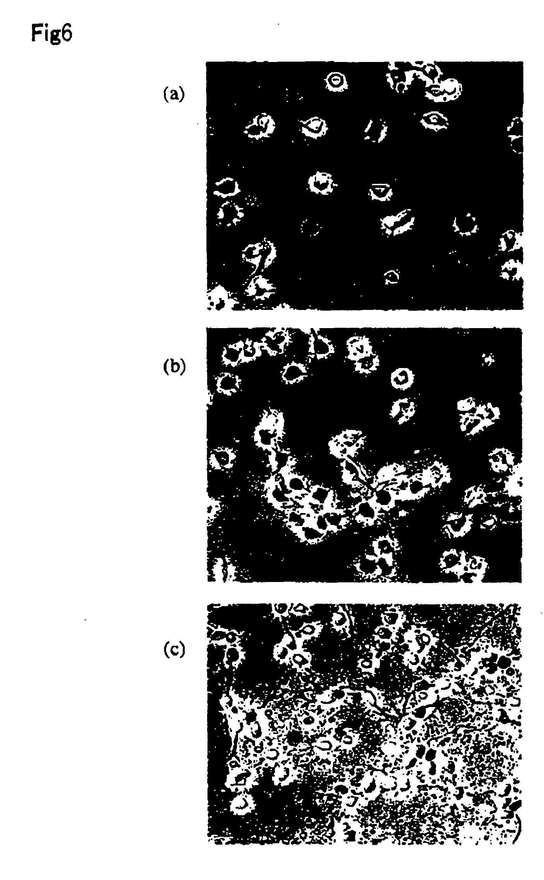

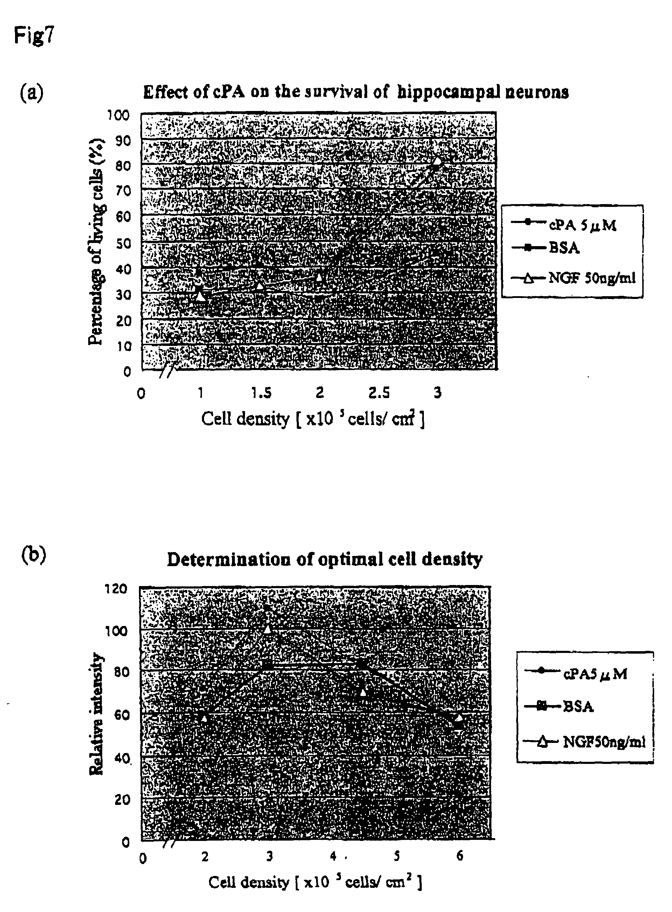

Firstly, 5µM of oleoyl cPA was added to the nerve cell culture solution under the condition as described in the above (B-1). As a result, the survival of the cells became better, and the elongation of neurites was also observed, as compared with the case of the control without cPA addition. The typical cells are shown in Fig. 6. Then, the relationship between the cell density and the survival rate was analyzed in more details. The cells were firstly inoculated at the cell density of 3.0x10⁵, 1.0x10⁵, 7.5x10⁴, and 5.0x10⁴ cells/cm², and their survival rates were compared with each other after 48 hours. As a result, the survival rates of the cells inoculated at the cell density of 3.0x10⁵ cells/cm² was the highest (Fig. 7(a)).

When the cell density was increased to 4.5x10⁵ or 6.0x10⁵ cells/cm², the survival rates of the cells were decreased as compared with the case of 3.0x10⁵ cells/cm² (Fig. 7(b)). Accordingly, it was revealed that the cell density of 3.0x10⁵ cells/cm² was suitable for cPA to increase most effectively the survival rate of the cells.

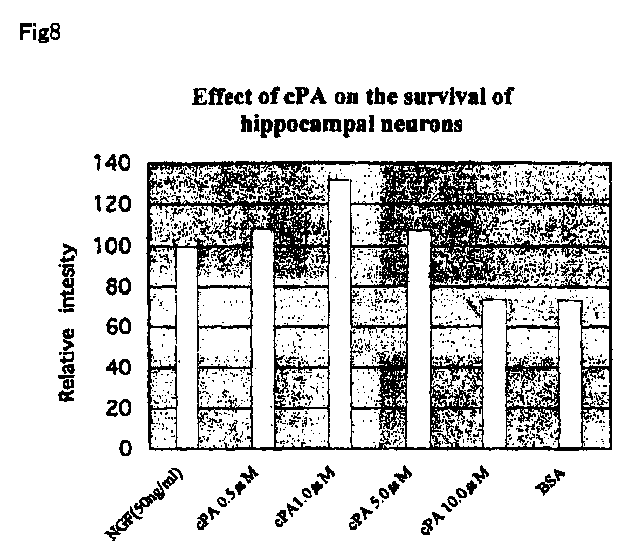

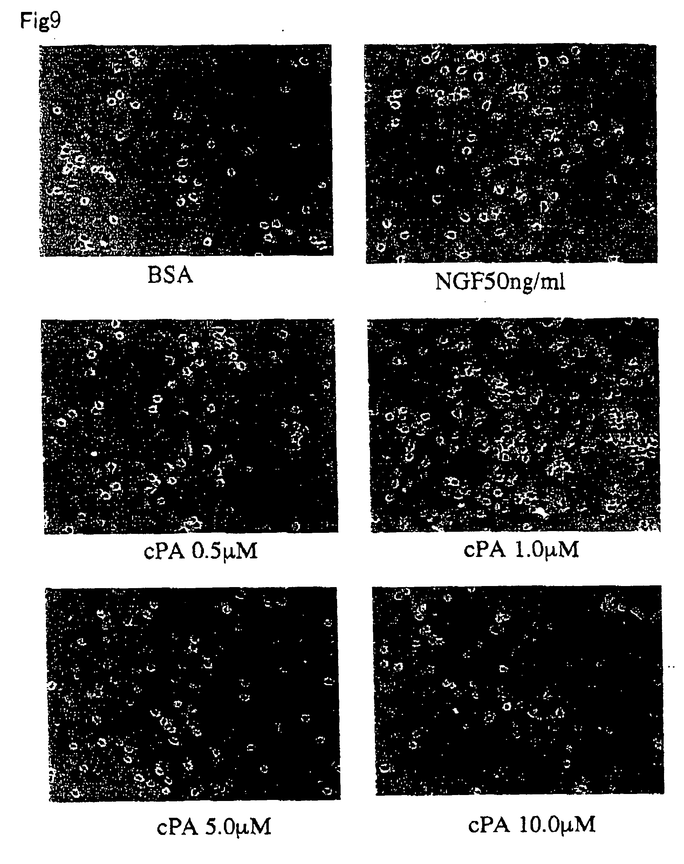

Next, in order to determine an optimal cPA level to increase the survival rate of the cell, 0.5, 1.0, 5.0, or 10.0µM of cPA was added to the culture after inoculation of the cells, and the survival of the cells was judged. As the result, it was revealed that the cPA was most effective for the cell survival at the level of 1.0µM (Figs. 8 and 9).

### (B-3) Action of cPA on the elongation of neurites

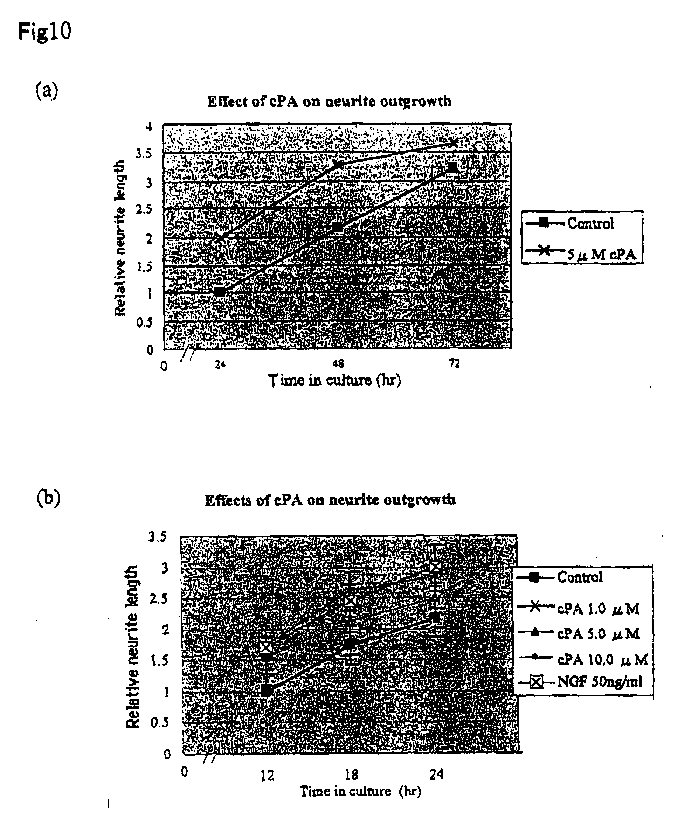

5µM of oleoyl cPA was added to the cell culture solution, and the cells were photographed after 24, 48, and 72 hours. The average length of neurites per cell was analyzed by the analysis with the image analysis software (NIH Image 1.62). As the result, after 24 hours, the average length in the case of adding cPA was observed to be twice longer than that of the control. The effect was strongest after 24 hours, as compared with those after 48 hours (1.5 times) and 72 hours (1.2 times) (Fig. 10(a)). Thus, it was considered that the evaluation of the action of cPA on the elongation of neurites at earlier time points was necessary, and the effect of cPA at various levels on the elongation of neurites was studied at 12, 18 and 24 hours after addition. As the result, at the time point after 12 hours, the elongation of neurites in the sample to which cPA of 1.0µM was added became about 1.7 times that of the control, and gave the maximum value (Fig. 10(b)). This value was comparable to that obtained by adding 50nm/ml of NGF (optimal level).

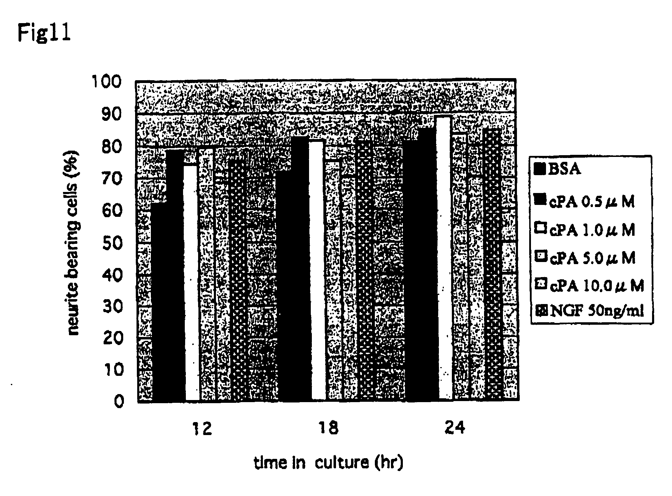

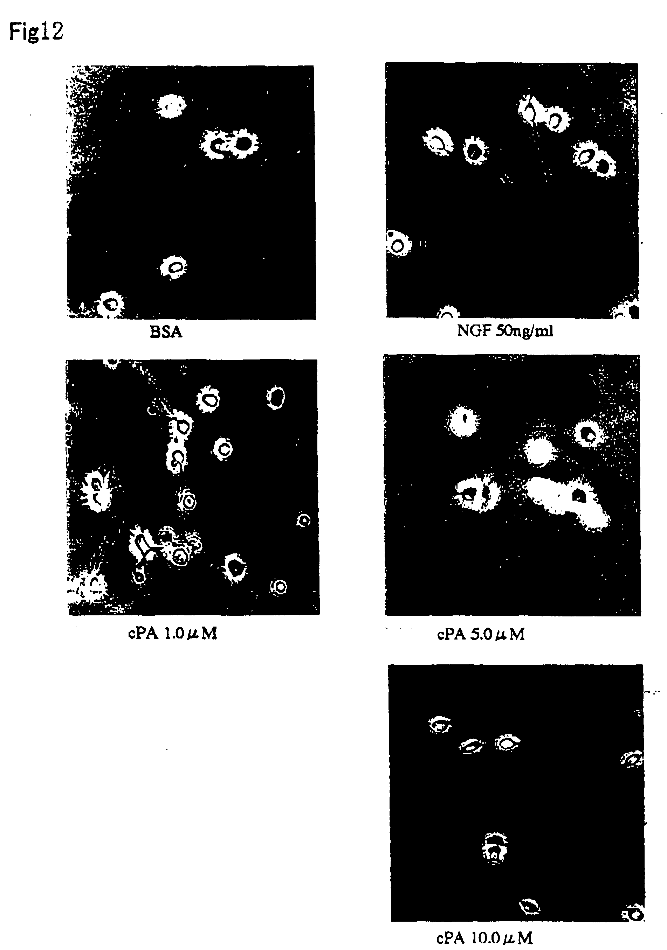

In the primary culture, not all the cells have neurites evenly. Cells with and without neurites exist together. Then, the effect of cPA on the ratio of cells having neurites was also analyzed. At 24 hours after addition, the ratio of cells having neurites was high in the sample to which cPA of 1.0µM was added. This value was high enough, as compared with that obtained by adding NGF (Figs. 11 and 12).

### (B-4) Analysis of the intracellular signal transduction system in the increase of survival rate by cPA

Wortmannin and LY294002, inhibitors against pbosphatidyl inositol 3-kinase (PI3K), were added to the culture medium respectively. Wortmannin lowered the survival rate nearly to the control level. LY294002 lowered it to the control value or lower.. In this case, many cells were destroyed and deformed. This result may be explained based on the appearance of cytotoxicity by LY294002.

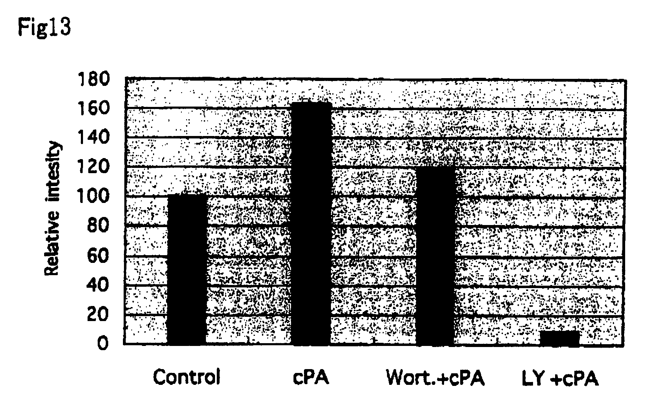

From the results above, it was suggested that the intramolecular signal transduction system mediated by the activity of PI3K is involved in the action of increasing the survival rate by cPA (Fig. 13).

### (C) Conclusion

The above results have revealed that cPA, in the level of 1.0µM, increases the survival rate of primary cultured nerve cells derived from a rat hippocampus and promotes the elongation of neurites. This test reveals especially that cPA has a nerve cell elongation action as a long term action. Therefore, cPA may promote the differentiation of the nerve cells in the long run.

### INDUSTRIAL APPLICABILITY

According to the present invention, it has been confirmed that a cPA derivative represented by the formula (I) increases the survival rate of the nerve cells derived from a mammalian hippocampus and promotes the elongation of the neurite. Therefore, it has been revealed that the cPA derivative represented by the formula (I) which is used in the present invention is useful as a therapeutic agent for nerve disease such as dementia, Alzheimer's disease, Alzheimer's senile dementia, amyotrophic lateral sclerosis, Parkinson's disease, cerebral stroke, cerebral infarction or head injury. According to the present invention, there is provided a medicament for treating and preventing a nerve disease, which is very effective for the prevention, treatment and rehabilitation of various diseases caused by death of the brain nerve cells, by increasing the survival rate of the nerve cells and promoting the elongation of neurites

## Claims

1. A medicament for promoting the survival of nerve cells, which comprises a cyclic phosphatidic acid derivative represented by the formula (I) as an active ingredient: 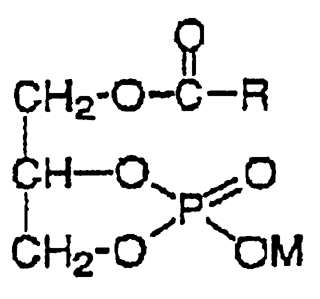 wherein R is a C₁₋₃₀ linear or branched alkyl group, a C₂₋₃₀ linear or branched alkenyl group, or a C₂₋₃₀ linear or branched alkynyl group, wherein these groups may contain a cycloalkane ring or an aromatic ring; and M is a hydrogen atom or a counter cation.

2. A medicament for promoting the elongation of nerve cells, which comprises a cyclic phosphatidic acid derivative represented by the formula (I) as an active ingredient: 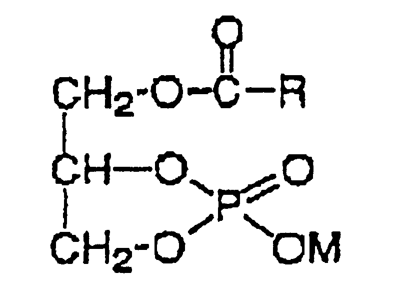 wherein R is a C₁₋₃₀ linear or branched alkyl group, a C₂₋₃₀ linear or branched alkenyl group, or a C₂₋₃₀ linear or branched alkynyl group, wherein these groups may contain a cycloalkane ring or an aromatic ring; and M is a hydrogen atom or a counter cation.

3. A medicament for treating and/or preventing a nerve disease, which comprises a cyclic phosphatidic acid derivative represented by the formula (I) as an active ingredient: 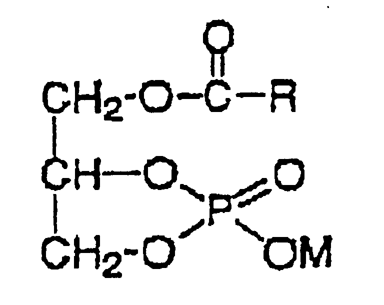 wherein R is a C₁₋₃₀ linear or branched alkyl group, a C₂₋₃₀ linear or branched alkenyl group, or a C₂₋₃₀ linear or branched alkynyl group, wherein these groups may contain a cycloalkane ring or an aromatic ring; and M is a hydrogen atom or a counter cation.

4. The medicament according to any of claims 1 to 3 wherein the nerve disease is selected from dementia, Alzheimer's disease, Alzheimer's senile dementia, amyotrophic lateral sclerosis, Parkinson's disease, cerebral stroke, cerebral infarction or head injury

5. The medicament according to any of claims 1 to 4 wherein the cyclic phosphatidic acid derivative represented by the formula (I) is 1-oleoyl cyclic phosphatidic acid.
